# FASCICULE DE BREVET EUROPEEN

(11) **EP 1 274 393 B1**
(45) Date de publication et mention de la délivrance du brevet: **05.07.2006**
(21) Numéro de dépôt: 01925617.1
(22) Date de dépôt: 11.04.2001
(51) Int. Cl.: A61Q 5/10, A61K 8/49, A61K 8/41, A61K 8/34

(54) **COMPOSITION DE TEINTURE D'OXYDATION DES FIBRES KERATINIQUES ET PROCEDE DE TEINTURE METTANT EN OEUVRE CETTE COMPOSITION**
OXIDATIONSFÄRBEMITTEL FÜR KERATINISCHE FASERN UND FÄRBUNGSVERFAHREN MIT DIESEM MITTEL
OXIDATION DYEING COMPOSITION FOR KERATINOUS FIBRES AND METHOD USING SAME

(30) Priorité: 12.04.2000 FR 0004717
(43) Date de publication de la demande: 15.01.2003
(73) Titulaire: L'ORÉAL, 75008 Paris (FR)
(72) Inventeur: AUDOUSSET, Marie-Pascale, F-92600 Asnières (FR)
(74) Mandataire: Fevrier, Murielle Françoise E.
(86) Numéro de dépôt international: PCT/FR2001/001110
(87) Numéro de publication internationale: WO 2001/076544

(56) Documents cités:
- EP-A- 0 166 155
- DE-A- 19 545 854
- DE-A- 19 610 946
- US-A- 4 473 375

## Description

L'invention a pour objet une composition pour la teinture d'oxydation des fibres kératiniques, et en particulier des fibres kératiniques humaines telles que les cheveux comprenant, dans un milieu approprié pour la teinture,
- à titre de premier coupleur au moins un dérivé de la 3,5-diaminopyridine choisi de façon appropriée ;
- et à titre de second coupleur au moins un méta-diphénol substitué et/ou l'un de ses sels d'addition avec un acide ;
- et au moins une base d'oxydation du type para-phénylènediamine et/ou l'un de ses sels d'addition avec un acide.

L'invention a également pour objet le procédé de teinture mettant en oeuvre cette composition.

L'invention a également pour objet le procédé de teinture mettant .en oeuvre cette composition.

Il est connu de teindre les fibres kératiniques et en particulier les cheveux humains avec des compositions tinctoriales contenant des précurseurs de colorant d'oxydation, en particulier des ortho- ou para-phénylènediamines, des ortho ou paraaminophénols, des bases hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces demiers étant choisis notamment parmi les méta-diamines aromatiques, les méta-aminophénols, les méta-diphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans les brevets US 4 473 375 et DE 31 32 885 des compositions de teinture d'oxydation contenant certains dérivés 3,5-diamino pyridine à titre de coupleur, en association avec des bases d'oxydation classiquement utilisées en teinture d'oxydation telles que par exemple des para-phénylènediamines, des para-aminophénols.. De telles compositions ne sont cependant pas toujours satisfaisantes, notamment du point de vue de la puissance et de la chromaticité des colorations obtenues.

Or, la demanderesse vient maintenant de découvrir, de façon totalement inattendue et surprenante, qu'il est possible d'obtenir de nouvelles teintures, capables de conduire à des colorations puissantes, particulièrement chromatiques et brillantes, peu sélectives, et présentant d'excellentes propriétés de résistances aux diverses agressions que peuvent subir les fibres kératiniques en associant un dérivé de 3,5-diamino pyridine de formule (I) définie ci-après à un deuxième coupleur du type méta-diphénol substitué et à au moins une base d'oxydation du type para-phénylènediamine.

Ces découvertes sont à la base de la présente invention.

L'invention a donc pour premier objet une composition pour la teinture d'oxydation des fibres kératiniques et en particulier des fibres kératiniques humaines telles que les cheveux, caractérisée par le fait qu'elle comprend, dans un milieu approprié pour la teinture :
- à titre de premier coupleur, au moins un dérivé de 3,5-diamino pyridine répondant à la formule générale suivante (I) : 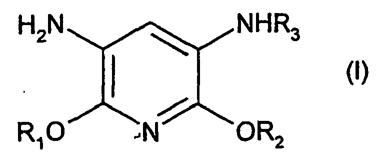
dans laquelle :
- R₁ et R₂, identiques ou différents, représentent un radical alkyle en C₁-C₄, monohydroxyalkyle en C₁-C₄, ou polyhydroxyalkyle en C₂-C₄,
- R₃ représente un atome d'hydrogène, un radical alkyle en C₁-C₄, monohydroxyalkyle en C₁-C₄ ou polyhydroxyalkyle en C₂-C₄ et/ou l'un de ses sels d'addition avec un acide ;
- et à titre de second coupleur, un méta-diphénol substitué et/ou l'un de ses sels d'addition avec un acide ;
- au moins une base d'oxydation du type para-phénylènediamine et/ou l'un de leurs sels d'addition avec un acide.

La composition tinctoriale conforme à l'invention conduit à des colorations puissantes, très chromatiques, et présentant d'excellentes propriétés de résistances à la fois vis à vis des agents atmosphériques tels que la lumière et les intempéries et vis à vis de la transpiration et des différents traitements que peuvent subir les cheveux.

L'invention a également pour objet un procédé de teinture d'oxydation des fibres kératiniques mettant en oeuvre cette composition tinctoriale.

Parmi les dérivés de 3,5-diamino pyridine de formule (1) conformes à l'invention, on peut citer la 2,6-diméthoxy-3,5-diaminopyridine, la 2,6-diéthoxy-3,5-diaminopyridine, la 2,6-di-(β-hydroxyéthyloxy)-3,5-diaminopyridine et leurs sels d'addition avec un acide.

Selon l'invention, la composition tinctoriale renferme de préférence de la 2,6-diméthoxy-3,5-diaminopyridine, et/ou au moins l'un de ses sels d'addition avec un acide.

Le ou les dérivés de 3,5-diaminopyridine de formule (I) utilisables à titre de premier coupleur dans la composition tinctoriale conforme à l'invention représentent de préférence de 0,0001 à 10 % en poids environ du poids total de la composition tinctoriale, et encore plus préférentiellement de 0,005 à 5 % en poids environ de ce poids.

Le ou les méta-diphénols substitués pouvant être utilisés à titre de deuxième coupleur dans la composition tinctoriale conforme à l'invention sont de préférence choisis parmi les composés de formule (II) suivante, et leurs sels d'addition avec un acide : 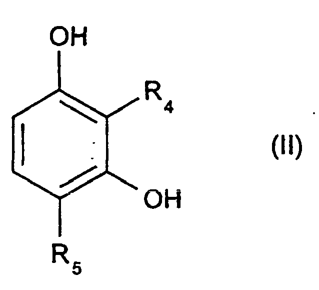 dans laquelle :
- R₄ et R₅, identiques ou différents, représente un atome d'hydrogène, un radical alkyle en C₁-C₄ ou un atome d'halogène choisi parmi le chlore, le brome ou le fluor ; étant entendu qu'au moins un des radicaux R₄ et R₅ est différent d'un atome d'hydrogène.

Parmi les méta-diphénols de formule (II) ci-dessus, on peut plus particulièrement citer le 2-méthyl 1,3-dihydroxy benzène, le 4-chloro 1,3-dihydroxy benzène, le 2-chloro 1,3-dihydroxybenzène, et leurs sels d'addition avec un acide.

Selon une forme de réalisation particulièrement préférée de l'invention, la composition tinctoriale renferme du 2-méthyl 1,3-dihydroxy benzène et/ou l'un de ses sels d'addition avec un acide.

Le ou les méta-diphénols substitués utilisables à titre de coupleur représentent de préférence de 0,0001 à 10 % en poids environ du poids total de la composition tinctoriale, et encore plus préférentiellement de 0,005 à 5 % en poids environ de ce poids.

Parmi les para-phénylènediamines utilisables à titre de base d'oxydation dans les composition tinctoriales conformes à l'invention, on peut notamment citer les composés de formule (III) suivante et leurs sels d'addition avec un acide : 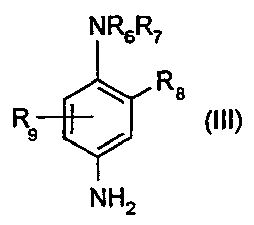 dans laquelle :
- R₆ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
- R₇ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ; ,
- R₈ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₉ représente un atome d'hydrogène, d'halogène ou un radical alkyle en C₁-C₄.

Parmi les groupements azotés de la formule (III) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les para-phénylènediamines de formule (III) ci-dessus, on peut plus particulièrement citer la para-phénylènediamine, la paratoluylènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénytènediamine, la N-(β-méthoxyéthyl) para-phénylènediamine, et leurs sels d'addition avec un acide.

Parmi les para-phénylènediamines de formule (III) ci-dessus, on préfère tout particulièrement la para-phénylènediamine, la paratoluylènediamine, la 2-isopropyl para-phénylènediamine, la 2-β-hydroxyéthyl para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-méthyl 1-N-(β-hydroxyéthyl) para-phénylènediamine et leurs sels d'addition avec un acide.

Les para-phénylènediamines représentent de préférence de 0,0005 à 12 % en poids environ du poids total de la composition tinctoriale conforme à l'invention, et encore plus préférentiellement de 0,005 à 6 % en poids environ de ce poids.

La composition tinctoriale conforme à l'invention peut également renfermer, en plus du ou des composés de formule (I) ci-dessus et de ou des méta-diphénols substitués, un ou plusieurs coupleurs additionnels pouvant être choisis parmi les coupleurs utilisés de façon classique en teinture d'oxydation et parmi lesquels on peut notamment citer les méta-aminophénols, les méta-phénylènediamines, la résorcine et les coupleurs hétérocycliques tels que par exemple les dérivés indoliques, les dérivés indoliniques, les dérivés pyridiniques autres que ceux de l'invention et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one , le 3-(4-hydroxy-1-méthyl-1H-indol-5-ylméthyl)-1-méthylpyridinium et leurs sels d'addition avec un acide.

Lorsqu'ils sont présents ces coupleurs additionnels représentent de préférence de 0,0001 à 10 % en poids environ du poids total de la composition tinctoriale et encore plus préférentiellement de 0,005 à 5 % en poids environ de ce poids.

En plus des para-phénylènediamines utilisées à titre de bases d'oxydation, la composition de teinture d'oxydation conforme à l'invention peut contenir une ou plusieurs bases d'oxydation additionnelles qui sont de préférence choisies parmi les bases d'oxydation classiquement utilisées en teinture d'oxydation et parmi lesquelles on peut notamment citer les bis-phénylalkylènediamines, les para-aminophénols, les ortho-aminophénols et les bases hétérocycliques.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques et les dérivés pyrazoliques.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demande de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazolé, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, le 4,5-diamino-1-β-hydroxyéthylpyrazole et leurs sels d'addition avec un acide.

La ou les bases d'oxydation additionnelles représentent de préférence de 0,0005 à 12 % en poids environ du poids total de la composition tinctoriale, et encore plus préférentiellement de 0,005 à 6 % en poids environ de ce poids.

D'une manière générale, les sels d'addition avec un acide utilisables dans le cadre des compositions tinctoriales de l'invention 3,5-diomino pyridinses (de formule (I), bases d'oxydation et coupleurs additionnels) sont notamment choisis parmi les chlorhydrates, les bromhydrates, les sulfates, les citrates, les succinates, les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, la propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les.produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (IV) suivante : 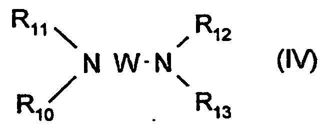 dans laquelle W est un reste propylène substitué ou non substitué par un groupement hydroxyle ou un radical alkyle en C₁-C₆ ; R₁₀, R₁₁, R₁₂ et R₁₃, identiques ou différents, représentent un atome d'hydrogène, un radical alkyle en C₁-C₆ ou hydroxyalkyle en C₁-C₆.

Les compositions de teinture d'oxydation conformes à l'invention peuvent également renfermer au moins un colorant direct, notamment pour modifier les nuances ou les enrichir en reflets.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un procédé de teinture d'oxydation des fibres kératiniques et en particulier des fibres kératiniques humaines telles que les cheveux, mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

Selon une forme de mise en oeuvre préférée du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, et les enzymes telles que les peroxydases, les laccases, les tyrosynases et les oxydo-réductases parmi lesquelles on peut en particulier mentionner les pyranose oxydases, les glucose oxydases, les glycérol oxydases, les lactates oxydases, les pyruvate oxydases, et les uricases.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui_est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

L'invention a enfin pour objet un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention.

| **EXEMPLES DE TEINTURE** | **1** | **2** | **3** |
|---|---|---|---|
| Dichlorhydrate de 2,6-diméthoxy-3,5-diamino pyridine (coupleur de formule (I)) | 0,242 | 0,242 | 0,726 |
| 2-méthyi 1,3-dihydroxybenzène | 0,248 | - | 0,124 |
| 4-chloro 1,3-dihydroxybenzène | - | 0,144 | - |
| Para-phénylènediamine (base d'oxydation) | 0,324 | - | - |
| Sulfate de N,N-bis-β-hydroxyéthylpara-phénylènediamine (base d'oxydation) | - | 0,624 | - |
| Dichlorhydrate de 2-hydroxyéthylpara-phénylènediamine (base d'oxydation) | - | - | 0,9 |
| Support de teinture commun n° | 1 | 2 | 1 |
| Eau déminéralisée qsp | 100 g | | 100 g |

### Support de teinture commun n°1 :

- Alcool oléique polyglycérolé à 2 moles de glycérol 4 g
- Alcool oléique polyglycérolé à 4 moles de glycérol ( 78 % M.A. ) 5,69 g M.A.
- Acide oléique 3,0 g
- Amine oléique 2 OE commercialisée sous la dénomination d'ETHOMEEN 012 par la société AKZO 7 g
- Laurylamino succinamate de diéthylaminopropyle,
- Sel de sodium à 55% M.A. 3 g M.A.
- Alcool oléique 5 g
- Diéthanolamide d'acide oléique 12 g
- Propylène glycol 3,5 g
- Alcool éthylique 7,0 g
- Dipropylène glycol 0,5 g
- Monométhyléther de propylène glycol 9 g
- Métabisulfite de sodium en solution aqueuse à 35% M.A. 0,455 g M.A.
- Acétate d'ammonium 0,8 g
- Antioxydant, séquestrant q.s.
- Parfum, conservateur q.s.
- Monoéthanolamine q.s.p. pH 9.8
- Colorants x g
- Eau déminéralisée q.s.p. 100 g

### Support de teinture commun n° 2 :

- Alcool oléique polyglycérolé à 2 moles de glycérol . 4 g
- Alcool oléique polyglycérolé à 4 moles de glycérol( 78 % M.A. ) 5,69 g M.A.
- Acide oléique 3,0 g
- Amine oléique 2 OE commercialisée sous la dénomination d'ETHOMEEN 012 par la société AKZO 7 g
- Laurylamino succinamate de diéthylaminopropyle,
- Sel de sodium à 55% M.A. 3 g M.A.
- Alcool oléique 5 g
- Diéthanolamide d'acide oléique 12 g
- Propylène glycol 3,5 g
- Alcool éthylique 7,0 g
- Dipropylène glycol 0,5 g
- Monométhyléther de propylène glycol 9 g
- Métabisulfite de sodium en solution aqueuse à 35% M.A. 0,455 g M.A.
- Acétate d'ammonium 0,8 g
- Antioxydant, séquestrant q.s.
- Parfum, conservateur q.s.
- Ammoniaque à 20% de NH₃ 10 g
- Colorants x g
- Eau déminéralisée q.s.p. 100 g

### MODE D'APPLICATION

La composition obtenue est mélangée, poids pour poids, avec de l'eau oxygénée titrant 20 volumes, et dont le pH est ajusté par une quantité précise d'acide orthophosphorique pur à 85% (2,5 g/ 100 g d'eau oxygénée) pour les exemples 1 et 3 et à pH =3 pour l'exemple 2. Le mélange est appliqué sur des cheveux gris à 90% de blancs, permanentés, à raison de 28 g pour 3 g de cheveux, pendant 30 mn. Les cheveux sont ensuite rincés, lavés avec un shampooing standard et séchés.

Les résultats sont indiqués dans le tableau suivant :

| **EXEMPLE** | **NUANCE OBTENUE** |
|---|---|
| **1** | Naturel cendré puissant |
| **2** | Bleu nuit puissant |
| **3** | Naturel cendré puissant |

## Revendications

1. Composition pour la teinture d'oxydation des fibres kératiniques et en particulier des fibres kératiniques humaines telles que les cheveux, **caractérisée par le fait qu'**elle comprend, dans un milieu approprié pour la teinture :
- à titre de premier coupleur, au moins un dérivé de 3,5-diamino pyridine répondant à la formule générale suivante (I) : 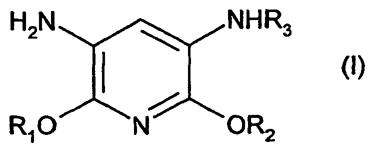
dans laquelle :
- R₁ et R₂, identiques ou différents, représentent un radical alkyle en C₁-C₄, monohydroxyalkyle en C₁-C₄, ou polyhydroxyalkyle en C₂-C₄,
- R₃ représente un atome d'hydrogène, un radical alkyle en C₁-C₄, monohydroxyalkyle en C₁-C₄ ou polyhydroxyalkyle en C₂-C₄ et/ou l'un de ses sels d'addition avec un acide ;
- et à titre de second coupleur au moins un méta-diphénol substitué et/ou l'un de ses sels d'addition avec un acide ;
- et au moins une base d'oxydation du type para-phénylènediamine et/ou l'un de ses sels d'addition avec un acide.

2. Composition selon la revendication 1, **caractérisée par le fait que** le ou les dérivés de 3,5-diamino pyridine de formule (I) sont choisis parmi la 2,6-diméthoxy-3,5-diaminopyridine, la 2,6-diéthoxy-3,5-diaminopyridine, la 2,6-di-(β-hydroxyéthyloxy)-3,5-diaminopyridine et leurs sels d'addition avec un acide.

3. Composition selon la revendication 1, **caractérisée par le fait que** le dérivé de 3,5-diamino pyridine de formule (I) est la 2,6-diméthoxy-3,5-diaminopyridine ou l'un de ses sels d'addition avec un acide.

4. Composition selon l'une quelconque des revendications 1 à 3, **caractérisée par** le fait le ou les dérivés de 3,5-diaminopyridine de formule (I) représentent de 0,0001 à 10 % en poids environ du poids total de la composition tinctoriale.

5. Composition selon l'une quelconque des revendications 1 à 4, **caractérisée par** le fait le ou les méta-diphénols substitués sont choisis parmi ceux répondant à la formule (II) suivante : 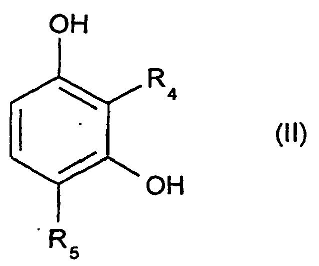 dans laquelle :
- R₄ et R₅, identiques ou différents, représente un atome d'hydrogène, un radical alkyle en C₁-C₄ ou un atome d'halogène choisi parmi le chlore, le brome ou le fluor ; étant entendu qu'au moins un des radicaux R₄ et R₅ est différent d'un atome d'hydrogène ; ainsi que leurs sels d'addition avec un acide.

6. Composition selon la revendication 5, où les méta-diphénols de formule (II) sont choisis parmi le 2-méthyl 1,3-dihydroxy benzène, le 4-chloro 1,3-dihydroxy benzène, le 2-chloro 1,3-dihydroxybenzène, et leurs sels d'addition avec un acide.

7. Composition selon l'une quelconque des revendications 1 à 6, **caractérisée par** le fait le ou les méta-diphénols représentent de 0,0001 à 10 % en poids environ du poids total de la composition tinctoriale.

8. Composition selon l'une quelconque des revendications 1 à 7, **caractérisée par** le fait le ou les para-phénylènediamines sont choisies parmi celles répondant à la formule (III) suivante : 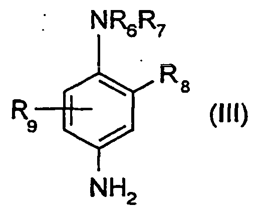 dans laquelle:
- R₆ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
- R₇ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ;
- R₈ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₉ représente un atome d'hydrogène, d'halogène ou un radical alkyle en C₁-C₄ et leurs sels d'addition avec un acide.

9. Composition selon la revendication 8, où la ou les para-phénylènediamines sont choisies parmi la para-phénylènediamine, la paratoluylènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la N-(β-méthoxyéthyl) para-phénylènediamine, la 2-méthyl 1-N-(β-hydroxyéthyl) para-phénylènediamine et leurs sels d'addition avec un acide.

10. Composition selon l'une quelconque des revendications 1 à 9, où la ou les para-phény!ènediamines représentent de 0,0005 à 12 % en poids environ du poids total de la composition tinctoriale.

11. Composition selon rune quelconque des revendications 1 à 10, **caractérisée par le fait qu'**elle renferme un ou plusieurs coupleurs additionnels différents des dérivés de 3,5-diaminopyridine de formule (I) et des méta-diphénols tels que définis dans les revendications précédentes.

12. Composition selon la revendication 11, où les coupleurs additionnels sont choisis parmi les méta-aminophénols, les méta-phénylènediamines, la résorcine et les coupleurs hétérocycliques et leurs sels d'addition avec un acide.

13. Composition selon la revendication 11 ou 12, où les coupleurs additionnels représentent de 0,0001 à 10 % en poids environ du poids total de la composition tinctoriale.

14. Composition selon l'une quelconque des revendications 1 à 10, **caractérisée par le fait qu'**elle renferme une ou plusieurs bases d'oxydation additionnelles autres que les para-phénylènediamines.

15. Composition selon la revendication 14, où les bases d'oxydation additionnelles sont choisies parmi les bis-phénylalkylènediamines, les para-aminophénols, les ortho-aminophénols et les bases hétérocycliques.

16. Composition selon la revendication 14 ou 15, où les bases d'oxydation additionnelles représentent de 0,0005 à 12 % en poids environ du poids total de la composition tinctoriale.

17. Composition selon l'une quelconque des revendications 1 à 16, **caractérisée par le fait que** les sels d'addition avec un acide sont choisis parmi les chlorhydrates, les bromhydrates, les sulfates et les tartrates, les lactates et les acétates.

18. Procédé de teinture des fibres kératiniques et en particulier des fibres kératiniques humaines telles que les cheveux, **caractérisé par le fait qu'**on applique sur lesdites fibres au moins une composition tinctoriale telle que définie à l'une quelconque des revendications 1 à 17, et que l'on révèle la couleur à pH acide, neutre ou alcalin à l'aidé d'un agent oxydant qui est ajouté juste au moment de l'emploi à la composition tinctoriale ou qui est présent dans une composition oxydante appliquée simultanément ou séquentiellement.

19. Procédé selon la revendication 18, **caractérisé par le fait que** l'agent oxydant présent dans la composition oxydante est choisi parmi le peroxyde d'hydrogène, le peroxyde d'urée, les bromates de métaux alcalins, les persels tels que les perborates, les percarbonates et persulfates, les peracides, et les enzymes.

20. Dispositif à plusieurs compartiments, ou "kit" de teinture à plusieurs compartiments, dont un premier compartiment renferme une composition tinctoriale telle que définie à l'une quelconque des revendications 1 à 17 et un second compartiment renferme une composition oxydante.

## Claims

1. Composition for the oxidation dyeing of keratinous fibres, and in particular of human keratinous fibres such as the hair, **characterized in that** it comprises, in a medium which is suitable for dyeing:
- as a first coupler, at least one 3,5-diaminopyridine derivative corresponding to the following general formula (1): 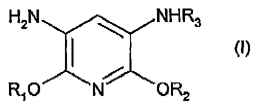
in which:
- R₁ and R₂, which may be identical or different, represent a C₁-C₄ alkyl radical, a C₁-C₄ monohydroxyalkyl radical or a C₂-C₄-polyhydroxyalkyl radical,
- R₃ represents a hydrogen atom, a C₁-C₄ alkyl radical, a C₁-C₄ monohydroxyalkyl radical or a C₂-C₄ polyhydroxyalkyl radical and/or one of its addition salts with an acid;
- and, as a second coupler, at least one substituted meta-diphenol and/or one of its addition salts with an acid;
- and at least one oxidation base of the para-phenylenediamine type and/or one of its addition salts with an acid.

2. Composition according to Claim 1, **characterized in that** the 3,5-diaminopyridine derivative(s) of formula (I) is (are) chosen from 2,6-dimethoxy-3,5-diaminopyridine, 2,6-diethoxy-3,5-diaminopyridine, 2,6-di(β-hydroxyethyloxy)-3,5-diaminopyridine and their addition salts with an acid.

3. Composition according to Claim 1, **characterized in that** the 3,5-diaminopyridine derivative of formula (I) is 2,6-dimethoxy-3,5-diaminopyridine or one of its addition salts with an acid.

4. Composition according to any one of Claims 1 to 3, **characterized in that** the 3,5-diaminopyridine derivative(s) of formula (I) represent(s) from 0.0001 to 10% by weight approximately of the total weight of the dye composition.

5. Composition according to any one of Claims 1 to 4, **characterized in that** the substituted meta-diphenol(s) is (are) chosen from those corresponding to the following formula (II): 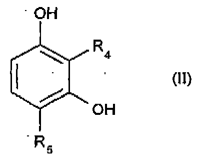 in which:
- R₄ and R₅, which may be identical or different, represent a hydrogen atom, a C₁-C₄ alkyl radical or a halogen atom chosen from chlorine, bromine or fluorine; it being understood that at least one of the radicals R₄ and R₅ is different from a hydrogen atom; and also their addition salts with an acid.

6. Composition according to Claim 5, in which the meta-diphenols of formula (II) are chosen from 2-methyl-1,3-dihydroxybenzene, 4-chloro-1,3-dihydroxybenzene, 2-chloro-1,3-dihydroxybenzene, and their addition salts with an acid.

7. Composition according to any one of Claims 1 to 6, **characterized in that** the meta-diphenol(s) represent(s) from 0.0001 to 10% by weight approximately of the total weight of the dye composition.

8. Composition according to any one of Claims 1 to 7, **characterized in that** the para-phenylenediamine(s) is (are) chosen from those corresponding to the following formula (III): 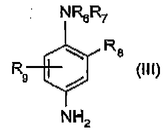 in which:
- R₆ represents a hydrogen atom, a C₁-C₄ alkyl radical, a C₁-C₄ monohydroxyalkyl radical, a C₂-C₄ polyhydroxyalkyl radical, a (C₁-C₄)alkoxy(C₁-C₄)alkyl radical or a C₁-C₄ alkyl radical substituted with a nitrogenous, phenyl or 4'-aminophenyl group;
- R₇ represents a hydrogen atom, a C₁-C₄ alkyl radical, a C₁-C₄ monohydroxyalkyl radical, a C₂-C₄ polyhydroxyalkyl radical, a (C₁-C₄)alkoxy(C₁-C₄)alkyl radical or a C₁-C₄ alkyl radical substituted with a nitrogenous group;
- R₈ represents a hydrogen atom, a halogen atom such as a chlorine, bromine, iodine or fluorine atom, a C₁-C₄ alkyl radical, a C₁-C₄ monohydroxyalkyl radical, a C₁-C₄ hydroxyalkoxy radical, an acetylamino(C₁-C₄)alkoxy radical, a C₁-C₄ mesylaminoalkoxy radical or a carbamoylamino(C₁-C₄)alkoxy radical,
- R₉ represents a hydrogen or halogen atom or a C₁-C₄ alkyl radical, and their addition salts with an acid.

9. Composition according to Claim 8, in which the para-phenylenediamine(s) is (are) chosen from para-phenylenediamine, para-tolylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine, 4-amino-N,N-bis(β-hydroxyethyl)-2-methylaniline, 4-amino-2-chloro-N,N-bis(β-hydroxyethyl)aniline, 2-β-hydroxyethyl-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N,N-(ethyl-β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, N-(β-methoxyethyl)-para-phenylenediamine and 2-methyl-1-N-(β-hydroxyethyl)-para-phenylenediamine, and their addition salts with an acid.

10. Composition according to any one of Claims 1 to 9, in which the para-phenylenediamine(s) represent(s) from 0.0005 to 12% by weight approximately of the total weight of the dye composition.

11. Composition according to any one of Claims 1 to 10, **characterized in that** it contains one or more additional couplers which are different from the 3,5-diaminopyridine derivatives of formula (I) and from the meta-diphenols as defined in the preceding claims.

12. Composition according to Claim 11, in which the additional couplers are chosen from meta-aminophenols, meta-phenylenediamines, resorcinol and heterocyclic couplers and their addition salts with an acid.

13. Composition according to Claim 11 or 12, in which the additional couplers represent from 0.0001 to 10% by weight approximately of the total weight of the dye composition.

14. Composition according to any one of Claims 1 to 10, **characterized in that** it contains one or more additional oxidation bases other than the para-phenylenediamines.

15. Composition according to Claim 14, in which the additional oxidation bases are chosen from bisphenylalkylenediamines, para-aminophenols, ortho-aminophenols and heterocyclic bases.

16. Composition according to Claim 14 or 15, in which the additional oxidation bases represent from 0.0005 to 12% by weight approximately of the total weight of the dye composition.

17. Composition according to any one of Claims 1 to 16, **characterized in that** the addition salts with an acid are chosen from hydrochlorides, hydrobromides, sulphates and tartrates, lactates and acetates.

18. Method for dyeing keratinous fibres, and in particular human keratinous fibres such as the hair, **characterized in that** at least one dye composition as defined in any one of Claims 1 to 17 is applied to said fibres, and **in that** the colour is developed at acidic, neutral or alkaline pH using an oxidizing agent which is added to the dye composition just at the time of use, or which is present in an oxidizing composition applied simultaneously or sequentially.

19. Method according to Claim 18, **characterized in that** the oxidizing agent present in the oxidizing composition is chosen from hydrogen peroxide, urea peroxide, alkali metal bromates, persalts such as perborates, percarbonates and persulphates, peracids and enzymes.

20. Multicompartment device, or multicompartment dyeing "kit", a first compartment of which contains a dye composition as defined in any one of Claims 1 to 17 and a second compartment of which contains an oxidizing composition.

## Patentansprüche

1. Zusammensetzung zum oxidativen Färben von Keratinfasern und insbesondere menschlichen Keratinfasern wie Haaren, **dadurch gekennzeichnet, dass** sie in einem zum Färben geeigneten Medium enthält:
- als ersten Kuppler mindestens ein 3,5-Diaminopyridinderivat der folgenden allgemeinen Formel (I): 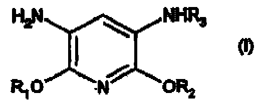
worin bedeuten:
- die Gruppen R₁ und R₂, die gleich oder verschieden sind, C₁₋₄-Alkyl, C₁₋₄-Monohydroxyalkyl oder C₂₋₄₋Polyhydroxyalkyl,
- R₃ ein Wasserstoffatom, C₁₋₄-Alkyl, C₁₋₄-Monohydroxyalkyl oder C₂₋₄-Polyhydroxyalkyl
und/oder eines seiner Additionssalze mit einer Säure;
- als zweiten Kuppler mindestens ein substituiertes meta-Dihydroxybenzol und/oder eines seiner Additionssalze mit einer Säure;
- und mindestens eine Oxidationsbase vom para-Phenylendiamintyp und/oder eines ihrer Additionssalze mit einer Säure.

2. Zusammensetzung nach Anspruch 1, **dadurch gekennzeichnet, dass** das oder die 3,5-Diaminopyridinderivat(e) der Formel (I) unter 2,5-Dimethoxy-3,5-diaminopyridin, 2,6-Diethoxy-3,5-diaminopyridin, 2,6-Di-(β-hydroxyethyloxy)-3,5-diaminopyridin und deren Additionssalzen mit einer Säure ausgewählt sind.

3. Zusammensetzung nach Anspruch 1, **dadurch gekennzeichnet, dass** das 3,5-Diaminopyridinderivat der Formel (I) das 2,6-Dimethoxy-3,5-diaminopyridin oder eines seiner Additionssalze mit einer Säure ist.

4. Zusammensetzung nach einem der Ansprüche 1 bis 3, **dadurch gekennzeichnet, dass** das oder die 3,5-Diaminopyridinderivat(e) der Formel (I) etwa 0,0001 bis 10 Gew.-% des Gesamtgewichts der Farbmittelzusammensetzung ausmachen.

5. Zusammensetzung nach einem der Ansprüche 1 bis 4, **dadurch gekennzeichnet, dass** das oder die substituierte(n) meta-Dihydroxybenzol(e) unter den Verbindungen der folgenden Formel (II) ausgewählt sind: 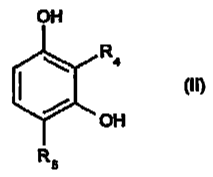 worin bedeuten:
- die Gruppen R₄ und R₅, die gleich oder verschieden sind, ein Wasserstoffatom, eine C₁₋₄-Alkylgruppe oder ein Halogenatom, das unter Chlor, Brom oder Fluor ausgewählt ist; mit der Maßgabe, dass mindestens eine der Gruppen R₄ und R₅ von Wasserstoff verschieden ist; sowie deren Additionssalze mit einer Säure.

6. Zusammensetzung nach Anspruch 5, wobei die meta-Dihydroxybenzole der Formel (II) unter 2-Methyl-1,3-dihydroxybenzol, 4-Chlor-1,3-dihydroxybenzol, 2-Chlor-1,3-dihydroxybenzol und deren Additionssalzen mit einer Säure ausgewählt sind.

7. Zusammensetzung nach einem der Ansprüche 1 bis 6, **dadurch gekennzeichnet, dass** das oder die meta-Dihydroxybenzol(e) etwa 0,0001 bis 10 Gew.-% des Gesamtgewichts der Farbmittelzusammensetzung ausmachen.

8. Zusammensetzung nach einem der Ansprüche 1 bis 7, **dadurch gekennzeichnet, dass** das oder die *p*-Phenylendiamin(e) unter den Verbindungen der folgenden Formel (III) ausgewählt sind: 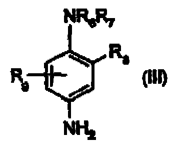 worin bedeuten:
- R₆ ein Wasserstoffatom, C₁₋₄-Alkyl, C₁₋₄-Monohydroxyalkyl, C₂₋₄-Polyhydroxyalkyl, Alkoxy(C₁₋₄)alkyl(C₁₋₄), eine mit einer Stickstoff-haltigen Gruppe substituierte C₁₋₄-Alkylgruppe, Phenyl oder 4'-Aminophenyl;
- R₇ ein Wasserstoffatom, C₁₋₄-Alkyl, C₁₋₄-Monohydroxyalkyl, C₂₋₄-Polyhydroxyalkyl, Alkoxy(C₁₋₄)alkyl(C₁₋₄), oder eine mit einer Stickstoff-haltigen Gruppe substituierte C₁₋₄-Alkylgruppe;
- R₈ ein Wasserstoffatom, ein Halogenatom, wie Chlor, Brom, Iod oder Fluor, C₁₋₄-Alkyl, C₁₋₄-Monohydroxyalkyl, C₁₋₄₋Hydroxyalkoxy, C₁₋₄-Acetylaminoalkoxy, C₁₋₄-Mesylaminoalkoxy oder C₁₋₄-Carbamoylaminoalkoxy;
- R₉ ein Wasserstoffatom, ein Halogenatom oder eine C₁₋₄-Alkylgruppe; und deren Additionssalze mit einer Säure.

9. Zusammensetzung nach Anspruch 8, wobei das oder die *p-*Phenylendiamin(e) unter *p*-Phenylendiamin, *p*-Toluylendiamin, 2-Chlor-*p*-phenylendiamin, 2,3-Dimethyl-*p*-phenylendiamin, 2,6-Dimethyl-*p*-phenylendiamin, 2,6-Diethyl-*p*-phenylendiamin, 2,5-Dimethyl-*p*-phenylendiamin, N,N-Dimethyl-*p*-phenylendiamin, N,N-Diethyl-*p*-phenylendiamin, N,N-Dipropyl-*p*-phenylendiamin, 4-Amino-N,N-diethyl-3-methylanilin, N,N-Bis-(β-hydroxyethyl)-*p-*phenylendiamin, 4-N,N-Bis-(β-hydroxyethyl)amino-2-methylanilin, la 4-N,N-Bis-(β-hydroxyethyl)amino-2-chloranilin, 2-β-Hydroxyethyl-*p*-phenylendiamin, 2-Fluor-*p*-phenylendiamin, 2-Isopropyl-*p*-phenylendiamin, N-(β-Hydroxypropyl)-*p*-phenylendiamin, 2-Hydroxymethyl-*p*-phenylendiamin, N,N-Dimethyl-3-methyl-*p*-phenylendiamin, N,N-(Ethyl, β-hydroxyethyl)-*p-*phenylendiamin, N-{β,γ-Dihydroxypropyl)-*p*-phenylendiamin, N-(4'-aminophenyl)-*p-*phenylendiamin, N-Phenyl-*p-*phenylendiamin, N-(β-Methoxyethyl)-*p*-phenylendiamin 2-Methyl-1-N-(β-hydroxyethyl)-*p-*phenylendiamin und deren Additionssalzen mit einer Säure ausgewählt sind.

10. Zusammensetzung nach einem der Ansprüche 1 bis 9, wobei das oder die *p*-Phenylendiamin(e) etwa 0,0005 bis 12 Gew.-% des Gesamtgewichts der Farbmittelzusammensetzung ausmachen.

11. Zusammensetzung nach einem der Ansprüche 1 bis 10, **dadurch gekennzeichnet, dass** sie einen oder mehrere ergänzende Kuppler enthält, die von den 3,5-Diaminopyridinderivaten der Formel (I) und den meta-Dihydroxybenzolen verschieden sind, wie sie in den vorhergehenden Ansprüchen definiert wurden.

12. Zusammensetzung nach Anspruch 11, wobei die ergänzenden Kuppler unter den meta-Aminophenolen, meta-Phenylendiaminen, Resorcin und heterocyclischen Kupplern und deren Additionssalzen mit einer Säure ausgewählt sind.

13. Zusammensetzung nach Anspruch 11 oder 12, wobei die ergänzenden Kuppler etwa 0,0001 bis 10 Gew.-% des Gesamtgewichts der Farbmittelzusammensetzung ausmachen.

14. Zusammensetzung nach einem der Ansprüche 1 bis 10, **dadurch gekennzeichnet, dass** sie ferner oder mehrere ergänzende Oxidationsbasen enthält, die von den *p*-Phenylendiaminen verschieden sind.

15. Zusammensetzung nach Anspruch 14, **dadurch gekennzeichnet, dass** die ergänzenden Oxidationsbasen unter den Bisphenylenalkylendiaminen, *p*-Aminophenolen, ortho-Aminophenolen und heterocyclischen Basen ausgewählt sind.

16. Zusammensetzung nach Anspruch 14 oder 15, wobei die ergänzenden Oxidationsbasen etwa 0,0005 bis 12 Gew.-% des Gesamtgewichts der Farbmittelzusammensetzung ausmachen.

17. Zusammensetzung nach einem der Ansprüche 1 bis 16, **dadurch gekennzeichnet, dass** die Additionssalze mit einer Säure unter den Hydrochloriden, Hydrobromiden, Sulfaten, Tartraten, Lactaten und Acetaten ausgewählt sind.

18. Verfahren zum Färben von Keratinfasern und insbesondere menschlichen Keratinfasern, wie Haaren, **dadurch gekennzeichnet, dass** auf die Fasern mindestens eine Farbmittelzusammensetzung nach einem der Ansprüche 1 bis 17 aufgetragen wird und die Farbe bei einem sauren, neutralen oder alkalischen pH-Wert mit einem Oxidationsmittel entwickelt wird, das bei der Anwendung zu der Farbmittelzusammensetzung gegeben wird oder das in einer oxidierenden Zusammensetzung gleichzeitig oder getrennt davon anschließend aufgetragen wird.

19. Verfahren nach Anspruch 18, **dadurch gekennzeichnet, dass** das Oxidationsmittel, das in der oxidierenden Zusammensetzung enthalten ist, unter Wasserstoffperoxid, Harnstoffperoxid, Alkalimetallbromaten, Salzen von Persäuren, wie Perboraten, Percarbonaten und Persulfaten, Persäuren und Enzymen ausgewählt ist.

20. Vorrichtung mit mehreren Abteilungen oder "Kit" zum Färben mit mehreren Abteilungen, wobei eine erste Abteilung eine Farbmittelzusammensetzung nach einem der Ansprüche 1 bis 17 und eine zweite Abteilung eine oxidierende Zusammensetzung enthält.
